# FASCICULE DE BREVET EUROPEEN

(11) **EP 4 643 099 B1**
(45) Date de publication et mention de la délivrance du brevet: **25.11.2026**
(21) Numéro de dépôt: 23838069.5
(22) Date de dépôt: 22.12.2023
(51) Int. Cl.: G01D 11/24, G01D 11/30, G01D 21/00, E03B 7/07, C02F 1/00, G01N 33/18, H05K 5/06

(54) **DISPOSITIF DE SURVEILLANCE ÉTANCHE**
ABGEDICHTETE ÜBERWACHUNGSVORRICHTUNG
SEALED MONITORING DEVICE

(30) Priorité: 26.12.2022 FR 2214500
(43) Date de publication de la demande: 05.11.2025
(73) Titulaire: Lacroix Group, 44800 Saint Herblain (FR)
(72) Inventeur: HOUDANT, Igor, 35770 VERN SUR SEICHE (FR); NAUDIN, Julien, 35770 VERN SUR SEICHE (FR); GOURON, Franck, 35770 VERN SUR SEICHE (FR)
(74) Mandataire: Lavoix
(86) Numéro de dépôt international: PCT/EP2023/087582
(87) Numéro de publication internationale: WO 2024/141455

(56) Documents cités:
- WO-A1-2022/058071
- GB-A- 2 547 463
- US-A1- 2019 120 666
- US-A1- 2019 301 957

## Description

La présente invention concerne un dispositif de surveillance, et plus particulièrement un dispositif de surveillance de réseaux d'eau.

Dans le domaine des réseaux d'eau, il est connu d'utiliser des dispositifs de surveillance pour surveiller le bon fonctionnement d'un réseau d'eau. Un tel dispositif de surveillance comprend généralement plusieurs éléments de connexion permettant sa connexion à des capteurs, ainsi qu'une carte électronique protégée dans un boîtier et embarquant un moyen de communication permettant la transmission des données issues des capteurs. En outre, le boîtier d'un tel dispositif de surveillance est généralement étanche, pour supporter son immersion. Par exemple, un dispositif de surveillance peut régulièrement être entièrement immergé lorsqu'il est disposé dans un regard d'un réseau d'eau pluviales, notamment en cas de fortes pluies.

La durée de vie d'un tel dispositif étant de plusieurs années, son vieillissement ne doit pas compromettre son étanchéité. En outre, il est nécessaire que son boîtier soit démontable, pour permettre l'accès à la carte électronique, notamment au cours d'opérations de maintenance.

Pour aboutir à une étanchéité complète du dispositif, il est connu de proposer un dispositif de surveillance de forme cylindrique, dont le boîtier est formé de deux parties vissées l'une à l'autre. Généralement, un joint torique est disposé entre les deux parties du boîtier pour assurer l'étanchéité du boîtier. Bien que donnant globalement satisfaction lorsque le dispositif de surveillance est en bon état, il est régulièrement constaté que l'ouverture d'un tel dispositif est particulièrement complexe après plusieurs années. En effet, l'exposition aux éléments et l'immersion d'un tel dispositif tend à amener le joint torique à coller les deux parties du boîtier entre elles, et tend à encrasser le pas de vis des deux parties du boîtier. Il est ainsi nécessaire de fournir un effort important pour dévisser les deux parties du boîtier, ce qui complexifie les opérations de maintenance.

Il est également connu de rendre étanche un dispositif de surveillance à l'aide de vis, qui viennent serrer deux parties du boîtier entre elles en compressant entre elles un joint d'étanchéité. Cette approche présente l'inconvénient de rendre le dispositif de surveillance plus onéreux à fabriquer, et augmente la complexité ainsi que la durée d'ouverture et de fermeture du boîtier. En outre, il est impossible d'ouvrir et de fermer le boîtier sans outils.

C'est à ces inconvénients qu'entend plus particulièrement remédier l'invention en proposant un dispositif de surveillance étanche qui est simple à ouvrir et à fermer tout au long de sa durée de vie, même après des immersions prolongées.

D'autres exemples connus de boîtier pour dispositifs de surveillance sont les suivants.

US 2019/301957 A1 divulgue un joint d'étanchéité compressé par montage forcé de l'élément de bâti supérieur dans l'élément de bâti inférieur, l'élément de bâti inférieur étant déformé autour de l'élément de bâti supérieur et du joint d'étanchéité.

US 2019/120666 A1 enseigne un double système d'encoches qui permet le crantage d'un élément de bâti sur l'autre et la compression d'un joint d'étanchéité.

WO 2022/058071 A1 propose une baïonnette qui permet de monter et de démonter facilement l'élément supérieur de boîtier sur l'élément inférieur, et d'exercer une pression pour l'étanchéité.

Dans GB 2 547 463 A enfin, la compression d'un élément sur l'autre et donc du joint d'étanchéité est maintenue grâce au montage d'une cheville dans des encoches correspondantes.

Pour remédier aux inconvénients mentionnés ci-dessus, l'invention concerne un dispositif de surveillance, comprenant un boîtier formant un volume intérieur et une carte électronique disposée dans le volume intérieur du boîtier. Le dispositif de surveillance est prévu pour être accroché sur un support. Le boîtier comporte une coque inférieure et une coque supérieure, juxtaposées selon un axe principal du dispositif de surveillance, la coque supérieure étant assemblée sur la coque inférieure.

Selon l'invention, le dispositif de surveillance comprend :
- un joint d'étanchéité, s'étendant dans un plan perpendiculaire à l'axe principal entre la coque inférieure et la coque supérieure,
- un système de fermeture, comportant au moins un ergot inférieur appartenant à la coque inférieure, au moins un ergot supérieur appartenant à la coque supérieure, au moins un organe de manœuvre, le ou chaque organe de manœuvre coopérant avec un ergot inférieur et avec un ergot supérieur.

Le ou chaque organe de manœuvre est actionnables entre :
- une position de verrouillage, dans laquelle le dispositif de surveillance est dans une configuration assemblée, la carte électronique étant alors protégée par le boîtier, et
- une position de déverrouillage, dans laquelle le dispositif de surveillance est dans une configuration non-assemblée, la carte électronique étant alors accessible.

L'actionnement de l'au moins un organe de manœuvre de la position de déverrouillage vers la position de verrouillage engendre un rapprochement de l'au moins un ergot inférieur et de l'au moins un ergot supérieur, entraînant une translation de la coque supérieure et de la coque inférieure l'une vers l'autre, selon l'axe principal, et entraînant la compression du joint d'étanchéité entre la coque supérieure et la coque inférieure.

Grâce à l'invention, la fermeture du boîtier du dispositif de surveillance est assurée de manière simple et fiable par les organes de manœuvre, et la translation des coques supérieure et inférieure l'une vers l'autre permet d'obtenir une compression du joint d'étanchéité garantissant une bonne étanchéité du dispositif de surveillance d'étanchéité.

Selon des aspects avantageux, mais non obligatoires de l'invention, ce dispositif de surveillance incorpore une ou plusieurs des caractéristiques suivantes, prises isolément ou selon toutes combinaisons techniquement admissibles :
- Le ou chaque organe de manœuvre comprend un logement de réception d'un premier ergot parmi l'ergot supérieur et l'ergot inférieur associés à cet organe de manœuvre, et une gorge de guidage du second ergot parmi l'ergot supérieur et l'ergot inférieur associés à cet organe de manœuvre. Le ou chaque organe de manœuvre est actionnable en rotation autour du premier ergot associé à cet organe de manœuvre, et le ou chaque organe de manœuvre est configuré pour transformer un mouvement de rotation de l'organe de manœuvre en un mouvement de translation du second ergot associé à cet organe de manœuvre selon l'axe principal.
- Le système de fermeture du dispositif de surveillance comprend au moins deux ergots inférieurs, au moins deux ergots supérieurs et au moins deux organes de manœuvre.
- Le dispositif de surveillance comprend deux organes de manœuvre et une poignée reliant les deux organes de manœuvre, et l'actionnement de la poignée entraîne l'actionnement des deux organes de manœuvre.
- La poignée est amovible, chaque organe de manœuvre présente une cavité de réception de la poignée, et la cavité de réception de chaque organe de manœuvre est configurée pour recevoir un éperon appartenant au support, permettant l'accrochage du dispositif de surveillance au support.
- Le joint d'étanchéité comprend au moins une barrière anti-infiltrations configurée pour empêcher l'infiltration d'eau entre la coque supérieure et la coque inférieure, et comprend une barrière anti-poussières, configurée pour empêcher le passage de poussières entre la coque supérieure et la coque inférieure.
- L'au moins une barrière anti-infiltrations est comprimée entre une face extérieure d'une première coque parmi la coque supérieure et la coque inférieure, la face extérieure étant dirigée à l'opposé du volume intérieur du boîtier, et une face intérieure de la deuxième coque parmi la coque supérieure et la coque inférieure, la face intérieure étant dirigée vers le volume intérieur du boîtier. La barrière anti-poussières est comprimée entre une face d'appui de la première coque et une extrémité de la deuxième coque, la face d'appui étant perpendiculaire à l'axe principal.
- La deuxième coque recouvre le joint d'étanchéité.
- Le dispositif de surveillance comprend un ou plusieurs éléments de connexion, chaque élément de connexion étant fixé au boîtier et connecté à la carte électronique. La coque inférieure comprend une extension, s'étendant selon l'axe principal à l'opposé de la coque supérieure, l'extension délimitant le volume intérieur du boîtier, les éléments de connexion étant fixés à l'extension, et une jupe, s'étendant selon l'axe principal à l'opposé de la coque supérieure, la jupe s'étendant au moins jusqu'à une paroi d'extrémité de l'extension, selon l'axe principal, la jupe entourant les éléments de connexion fixés à l'extension. De plus, l'extension et la jupe forment entre elles un volume complémentaire d'étanchéité, les éléments de connexion étant disposés dans le volume complémentaire d'étanchéité.
- La jupe présente des moyens d'accrochage, les moyens d'accrochage étant configurés pour permettre l'accrochage du dispositif de surveillance au support et pour empêcher une translation du dispositif de surveillance, par rapport au support, perpendiculairement à l'axe principal, et empêcher une translation du dispositif de surveillance, par rapport au support, dans une direction parallèle à l'axe principal et orientée de la coque supérieure vers la coque inférieure. De préférence, le dispositif de surveillance est configuré pour que l'accrochage du dispositif de surveillance sur le support ne soit possible que lorsque les organes de manoeuvre sont en position de verrouillage.
- La coque supérieure présente des moyens de blocage, configurés pour coopérer avec le support de sorte à empêcher une translation du dispositif de surveillance, par rapport au support, dans une direction parallèle à l'axe principal et orientée de la coque inférieure vers la coque supérieure.

Selon un autre aspect, la demande concerne un mode de réalisation dans lequel on prévoit que le dispositif de surveillance est dépourvu de système de fermeture tel que décrit ci-dessus, ou dans lequel le système de fermeture tel que décrit ci-dessus est facultatif. Dans ce mode de réalisation particulier, toute caractéristique décrite pour l'invention peut être prévue par ailleurs. Dans ce mode de réalisation particulier, le dispositif de surveillance comprend un boîtier formant un volume intérieur, une carte électronique disposée dans le volume intérieur du boîtier, et un ou plusieurs éléments de connexion, chaque élément de connexion étant fixé au boîtier et connecté à la carte électronique. Le boîtier comprend une coque inférieure et une coque supérieure, juxtaposées selon un axe principal du dispositif de surveillance, la coque supérieure étant assemblée sur la coque inférieure. En outre, la coque inférieure comprend une extension, s'étendant selon l'axe principal à l'opposé de la coque supérieure, l'extension délimitant le volume intérieur du boîtier, les éléments de connexion étant fixés à l'extension, et une jupe, s'étendant selon l'axe principal à l'opposé de la coque supérieure, la jupe s'étendant au moins jusqu'à une paroi d'extrémité de l'extension, selon l'axe principal, la jupe entourant les éléments de connexion fixés à l'extension. L'extension et la jupe forment entre elles un volume complémentaire d'étanchéité, les éléments de connexion étant disposés dans le volume complémentaire d'étanchéité.

L'invention sera mieux comprise et d'autres avantages de celle-ci apparaitront plus clairement à la lumière de la description qui va suivre d'un mode de réalisation d'un dispositif de surveillance donnée uniquement à titre d'exemple et faite en référence aux dessins annexés dans lesquels :
[Fig. 1] La figure 1 est une vue en perspective d'une installation comprenant un dispositif de surveillance conforme à l'invention.
[Fig. 2] La figure 2 est une vue en perspective éclatée de l'installation de la figure 1.
[Fig. 3] La figure 3 est une vue en perspective éclatée du dispositif de surveillance appartenant à l'installation des figures 1 et 2.
[Fig. 4] La figure 4 est une vue en perspective éclatée du dispositif de surveillance des figures 1 à 3, dans une configuration non-assemblée.
[Fig. 5] La figure 5 est une vue en coupe du dispositif de surveillance des figures 1 à 4, dans une configuration assemblée, selon le plan V de la figure 2, dans laquelle deux organes de manoeuvre du dispositif de surveillance sont masqués.
[Fig. 6] La figure 6 est une vue du détail VI de la figure 5.
[Fig. 7] La figure 7 est une vue de face d'un organe de manoeuvre du dispositif de surveillance des figures 3 à 6.

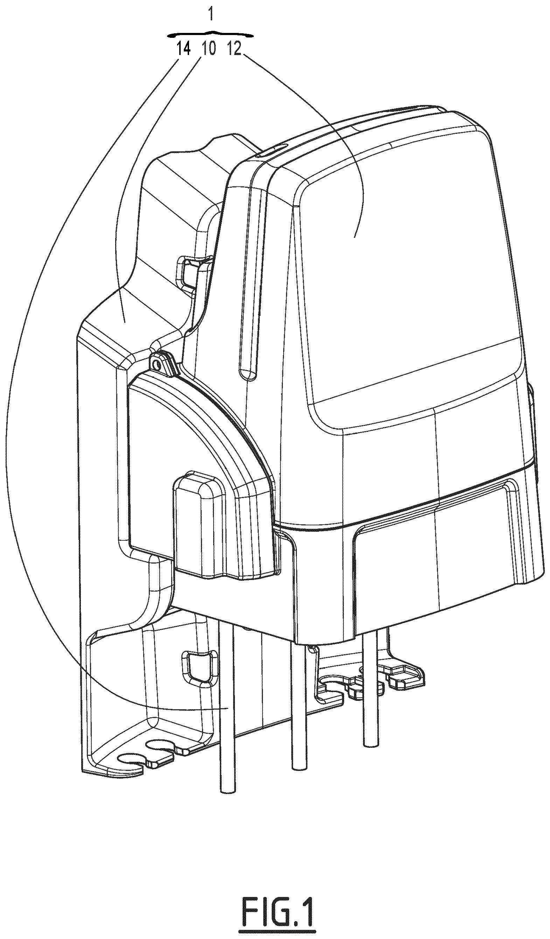

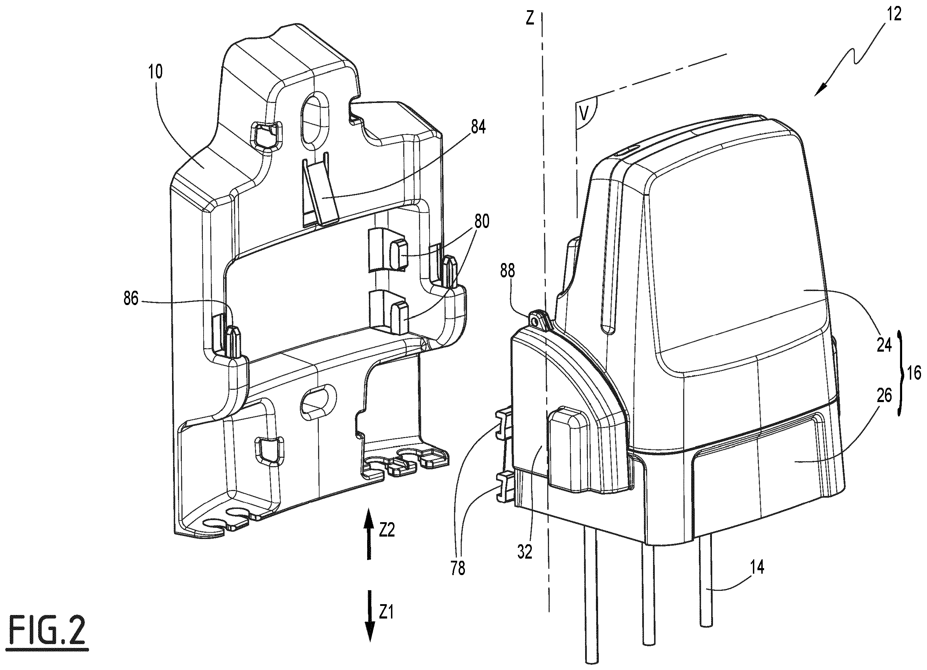

Une installation I est représentée sur les figures 1 et 2. Cette installation I comprend un support 10, un dispositif de surveillance 12 et plusieurs capteurs ou actionneurs 14.

L'installation I est par exemple une installation destinée à la gestion et à la surveillance d'un réseau d'eau, comme par exemple un réseau d'eau potable, un réseau d'eaux usées ou un réseau d'eaux pluviales. Dans des modes de réalisation, l'installation est destinée à être installée sous terre, par exemple dans un regard.

Les capteurs ou actionneurs 14 sont connectés au dispositif de surveillance 12. Le dispositif de surveillance 12 et les capteurs ou actionneurs 14 coopèrent pour récolter des données de fonctionnement du réseau, tel que par exemple des mesures de débit ou de pression, et/ou pour agir sur le fonctionnement du réseau, par exemple en actionnant des vannes. Les capteurs ou actionneurs 14 sont généralement déportés du dispositif de surveillance 12, et sont ici représentés uniquement par des câbles connectés au dispositif de surveillance 12.

Le support 10 est solidaire d'un bâti non-représenté du réseau d'eau, tel que par exemple un mur ou un poteau. Le support 10 permet la fixation du dispositif de surveillance 12. En pratique, le dispositif de surveillance 12 est monté de manière amovible sur le support 10, c'est-à-dire qu'il peut être démonté, par exemple pour permettre des opérations de maintenance.

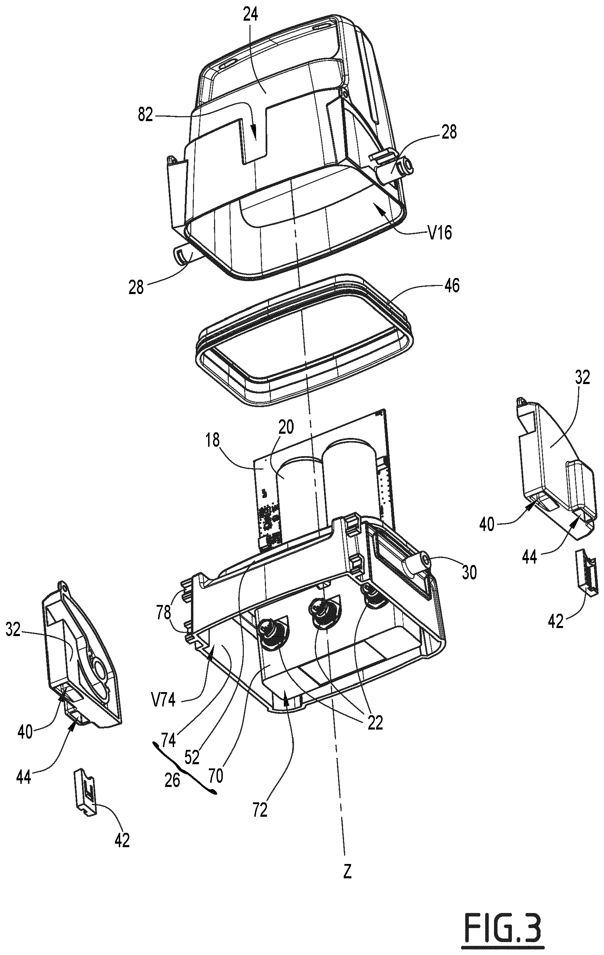

Le dispositif de surveillance 12 est représenté seul sur les figures 3 à 6 et comprend un boîtier 16, qui définit un volume intérieur V16.

Le dispositif de surveillance 12 comprend une carte électronique 18, disposée dans le volume intérieur V16 du boîtier 16. La carte électronique 18 est prévue pour collecter les données provenant des capteurs ou actionneurs 14, pour traiter ces données, et éventuellement pour communiquer ces données avec un élément externe à l'installation I, tel que par exemple un serveur distant. Pour permettre cette communication, la carte électronique 18 comprend de préférence un émetteur sans fil, relié tel que par exemple à une antenne Bluetooth ou encore à une antenne associée à un réseau de télécommunications mobiles.

De préférence, la carte électronique 18 comprend également un élément de stockage d'énergie électrique 20, tel qu'une pile ou une batterie, alimentant en énergie électrique le dispositif de surveillance 12.

Le dispositif de surveillance 12 comprend au moins un élément de connexion 22, dans l'exemple trois éléments de connexion 22. Chaque élément de connexion 22 est fixé sur le boîtier 16, et traverse le boîtier 16 de sorte à être connecté, d'une part, à la carte électronique 18, dans le volume intérieur V16, et, d'autre part, aux capteurs ou actionneurs 14 situés à l'extérieur du boîtier 16. En d'autres termes, les capteurs ou actionneurs 14 sont connectés à la carte électronique 18 par l'intermédiaire des éléments de connexion 22, qui traversent le boîtier 16.

Le boîtier 16 comporte une coque supérieure 24 et une coque inférieure 26 qui sont juxtaposées selon un axe principal Z du dispositif de surveillance, la coque supérieure 24 étant assemblée sur la coque inférieure. Ici, lorsque le dispositif de surveillance 12 est fixé au support 10, l'axe principal Z est vertical et la coque supérieure 24 est située au-dessus de la coque inférieure 26.

Dans la suite de la description, les orientations « haute », « basse », « supérieure », « inférieure » s'entendent relativement à l'axe principal Z et relativement à l'orientation présentée sur les figures, et non pas relativement à l'orientation réelle du dispositif de surveillance 12.

De préférence, le boîtier 16 présente une section essentiellement rectangulaire, considérée perpendiculairement à l'axe principal Z. Cette section essentiellement rectangulaire est avantageuse pour optimiser les dimensions du dispositif de surveillance 12, car un boîtier à section rectangulaire est généralement compact, en présentant moins d'espaces vides dans le volume intérieur V16, comparativement à un boîtier à section circulaire tel que connu dans l'état de la technique.

Les coques 24 et 26 sont assemblées entre elles de manière démontable, permettant ainsi l'ouverture du boîtier 16 et l'accès aux éléments internes, en particulier à la carte électronique 18, par exemple en vue d'opérations de maintenance. Dans le mode de réalisation illustré, et comme visible à la figure 3, la carte électronique 18 est fixée à la coque inférieure 26 du boîtier 16.

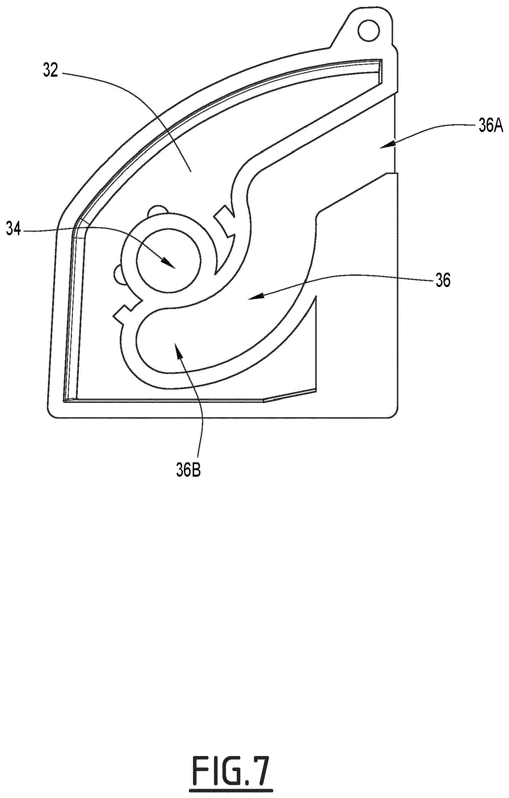

Pour permettre le montage et le démontage aisé des coques 24 et 26, le dispositif de surveillance 12 comprend un système de fermeture qui comporte deux ergots supérieurs 28, appartenant à la coque supérieure 24, deux ergots inférieurs 30, appartenant à la coque inférieure 26, et deux organes de manœuvre 32. Le détail d'un organe de manœuvre 32 est visible à la figure 7.

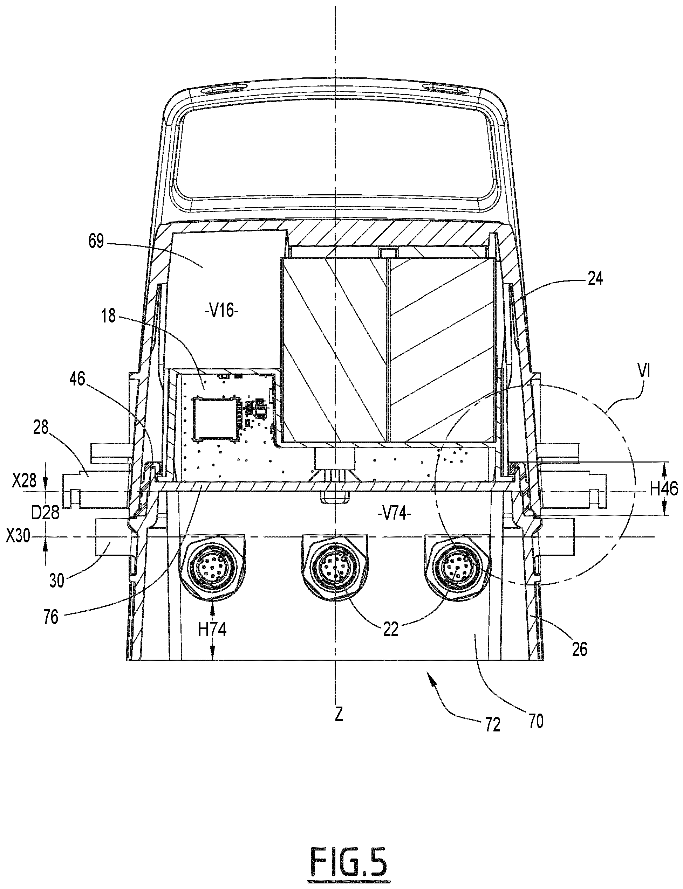

Ici, les deux ergots supérieurs 28 de la coque supérieure 24 s'étendent depuis deux faces opposées de la coque supérieure 24, selon un même axe X28, mieux visible à la figure 5. De même, les deux ergots inférieurs 30 de la coque inférieure 26 s'étendent depuis deux faces opposées de la coque inférieure, selon un même axe X30, mieux visible à la figure 5. Les axes X28 et X30 sont avantageusement perpendiculaires à l'axe principal Z. En pratique, chaque ergot supérieur 28 est situé à proximité d'un ergot inférieur 30, c'est-à-dire que chaque ergot supérieur 28 est aligné avec un ergot inférieur 30, selon l'axe principal Z, et séparé de cet ergot inférieur d'une distance D28 faible lorsque le boîtier 16 est fermé, de préférence inférieure à 40 mm, la distance D28 étant mesurée parallèlement à l'axe principal Z. Dans l'exemple, la distance D28 est égale à 15 mm.

Chaque organe de manœuvre 32 coopère avec l'un des deux ergots supérieurs 28 et avec l'ergot inférieur 30 situé à proximité dudit ergot supérieur et les deux organes de manœuvre 32 sont actionnables entre une position de verrouillage, dans laquelle le dispositif de surveillance 12 est dans une configuration assemblée, également dite configuration fermée, et une position de déverrouillage, dans laquelle le dispositif de surveillance est dans une configuration non-assemblée, également dite configuration ouverte. Avantageusement, l'ouverture et la fermeture du boîtier 16 s'effectue en une seule étape consistant à actionner simultanément les deux organes de manœuvre 32.

Lorsque le dispositif de surveillance 12 est dans une configuration assemblée, les deux coques 24 et 26 sont assemblées l'une à l'autre et la carte électronique 18 est alors protégée à l'intérieur du boîtier 16. Lorsque le dispositif de surveillance 12 est dans une configuration non-assemblée, les deux coques 24 et 26 ne sont pas assemblées l'une à l'autre et le boîtier 16 peut alors être ouvert, permettant l'accès à la carte électronique 18.

En pratique, l'actionnement des deux organes de manœuvre 32 de leur position de déverrouillage vers leur position de verrouillage entraîne un rapprochement des deux ergots 30 inférieurs et des deux ergots 28 supérieurs, par une translation de la coque supérieure 24 et de la coque inférieure 26 l'une vers l'autre, selon l'axe principal Z.

Dans l'exemple, et comme mieux visible à la figure 7, chaque organe de manœuvre 32 comprend à cet effet un logement 34, prévu pour recevoir l'ergot supérieur 28 correspondant, et une gorge 36, prévue pour recevoir l'ergot inférieur 30 correspondant.

Pour chaque organe de manœuvre 32, les dimensions du logement 34 sont sensiblement équivalentes aux dimensions de l'ergot supérieur 28, de sorte que les deux organes de manœuvre 32 sont mobiles en rotation autour des ergots supérieurs 28, c'est-à-dire autour des axes X28.

La gorge 36 de chaque organe de manœuvre 32 présente une entrée 36A et un fond 36B. Lorsque les organes de manœuvre 32 sont en position de verrouillage, chaque ergot inférieur 30 est situé dans le fond 36B de la gorge 36 de l'organe de manœuvre correspondant, et lorsque les organes de manœuvre 32 sont en position de déverrouillage, chaque ergot inférieur 30 est situé dans l'entrée 36A de la gorge 36 de l'organe de manœuvre correspondant. Ainsi, l'actionnement des organes de manœuvre 32 autour des ergots supérieurs 28 entraîne un déplacement des ergots inférieurs 30 dans les gorges 36.

En outre, les gorges 36 sont prévues de sorte que la rotation des organes de manœuvre 32 de leur position de déverrouillage vers leur position de verrouillage entraîne une translation des ergots inférieurs 30 vers les ergots supérieurs 28, c'est-à-dire un rapprochement des ergots inférieurs et des ergots supérieurs, selon l'axe principal Z. Les ergots inférieurs 30 et supérieurs 28 appartenant respectivement à la coque inférieure 26 et à la coque supérieure 24, leur rapprochement selon l'axe principal Z entraîne le rapprochement des coques supérieure et inférieure l'une de l'autre selon l'axe principal Z.

En d'autres termes, l'actionnement des organes de manœuvre 32 de leur position de déverrouillage vers leur position de verrouillage, ici en rotation, entraîne une translation de la coque inférieure 26 vers la coque supérieure 24 selon l'axe principal Z, ce qui permet de fermer le boîtier 16. De manière particulièrement avantageuse, les organes de manœuvre 32 fonctionnent comme des démultiplicateurs permettant d'augmenter l'effort s'exerçant sur le boîtier 16 lors de sa fermeture, comparativement à une fermeture directe du boîtier par un rapprochement manuel des deux coques 24, 26. En effet, les organes de manœuvre 32 convertissent un mouvement de rotation d'amplitude importante en un mouvement de translation des ergots 28, 30 d'amplitude faible.

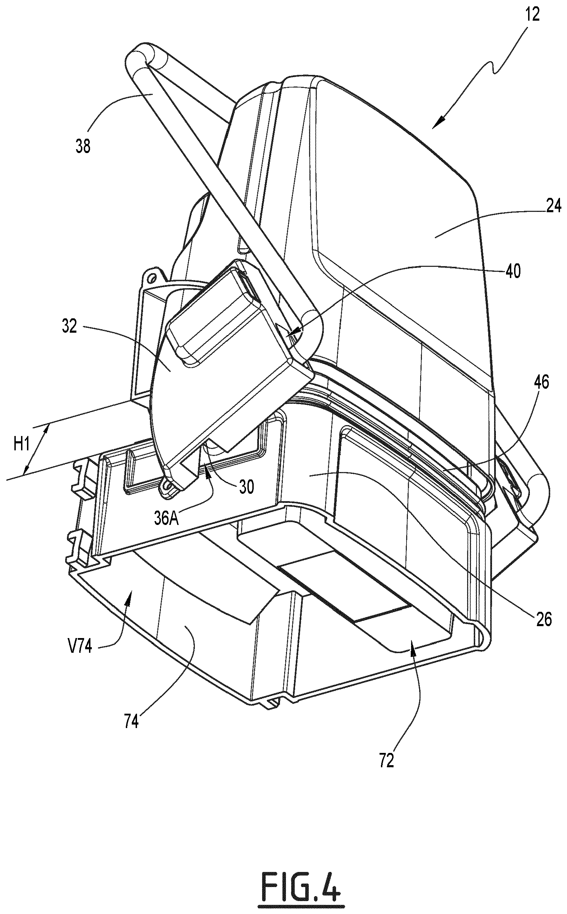

Avantageusement, le dispositif de surveillance 12 comprend une poignée 38 et chaque organe de manœuvre 32 présente une cavité de réception 40 de la poignée 38. Comme mieux visible à la figure 4, la poignée 38 présente deux extrémités s'étendant chacune dans une cavité de réception 40 de l'un des deux organes de manœuvre. La poignée 38 est prévue pour que son actionnement entraîne l'actionnement des deux organes de manœuvre 32, en rotation autour de l'axe X28. Ainsi, la poignée 38 facilite l'actionnement des organes de manœuvre 32, en permettant de bénéficier d'un effort de bras de levier plus important pour actionner les organes de manœuvre, et en permettant un actionnement simultané des deux organes de manœuvre. En outre, la poignée 38 est avantageusement amovible, de sorte qu'une fois le dispositif de surveillance 12 fermé, il ne soit pas nécessaire de conserver la poignée montée dans les cavités de réception 40. En pratique, dans l'exemple, la poignée 38 est simplement insérée dans les cavités de réception 40.

L'amplitude de la translation des coques supérieure 24 et inférieure 26 l'une vers l'autre est égale à une hauteur H1 séparant la coque supérieure de la coque inférieure lorsque les ergots inférieurs 30 sont localisés à l'entrée 36A des gorges 36, c'est-à-dire dans la position représentée à la figure 4. Dans l'exemple, la hauteur H1 est égale à 15 mm.

Dans l'exemple, chaque organe de manœuvre 32 est assemblé sur l'ergot supérieur 28 correspondant en étant emboîté sur cet ergot supérieur puis est maintenu en position à l'aide d'un élément de verrouillage 42, qui est inséré dans une ouverture 44 de l'organe de manœuvre et qui coopère avec l'ergot supérieur 28 pour empêcher le démontage de l'organe de manœuvre. Ainsi, les organes de manœuvre 32 sont prévus pour être fixés à la coque supérieure 24 lors du premier assemblage du dispositif de surveillance 12 et ne sont pas prévus pour être démontés ensuite. En pratique, il est toutefois possible de démonter les éléments de verrouillage 42 à l'aide d'un outil pour permettre le démontage des organes de manœuvre 32.

Pour assurer l'étanchéité du dispositif de surveillance 12 et éviter l'entrée d'eau dans le volume intérieur V16 du boîtier 16 au niveau de la jonction entre la coque supérieure 24 et la coque inférieure 26, le dispositif de surveillance comprend un joint d'étanchéité 46.

Le joint d'étanchéité 46 s'étend dans un plan perpendiculaire à l'axe principal Z, entre la coque supérieure 24 et la coque inférieure 26, et est comprimé entre la coque supérieure et la coque inférieure lorsque le dispositif de surveillance est dans sa configuration assemblée, c'est-à-dire lorsque les organes de manœuvre 32 sont en position de verrouillage.

En outre, le joint d'étanchéité 46 est accroché à l'une des deux coques 24, 26, dans l'exemple à la coque inférieure 26, de sorte à être maintenu en position de manière contrôlée au cours de la fermeture du boîtier 16. Ainsi, lorsque les organes de manœuvre 32 sont manœuvrés pour fermer le boîtier 16, le joint d'étanchéité 46 est comprimé sans risque d'être mal positionné. Pour cela, le joint d'étanchéité comporte une paroi principale 48 et un rebord 50, qui forment entre eux une gorge dans laquelle pénètre une bordure supérieure 52 de la coque inférieure 26. En variante non-représentée de l'invention, le joint d'étanchéité 46 ne comprend pas de rebord 50 et le joint d'étanchéité est fixé autrement à la coque inférieure 26, par exemple en étant collé à la bordure supérieure 52.

Avantageusement, le joint d'étanchéité 46 est comprimé entre une face extérieure 54 de la coque inférieure 26, qui correspond en pratique à une face extérieure de la bordure supérieure 52, et une face intérieure 56 de la coque supérieure 24. La face extérieure 54 est dirigée à l'opposé du volume intérieur V16 du boîtier 16 et la face intérieure 56 est dirigée vers le volume intérieur du boîtier. De préférence, les face extérieure 54 et intérieure 56 sont parallèles l'une à l'autre, et un angle α formé entre une direction normale N56 aux face extérieure et intérieure est compris entre 90° et 100°, de préférence égal à 93°.

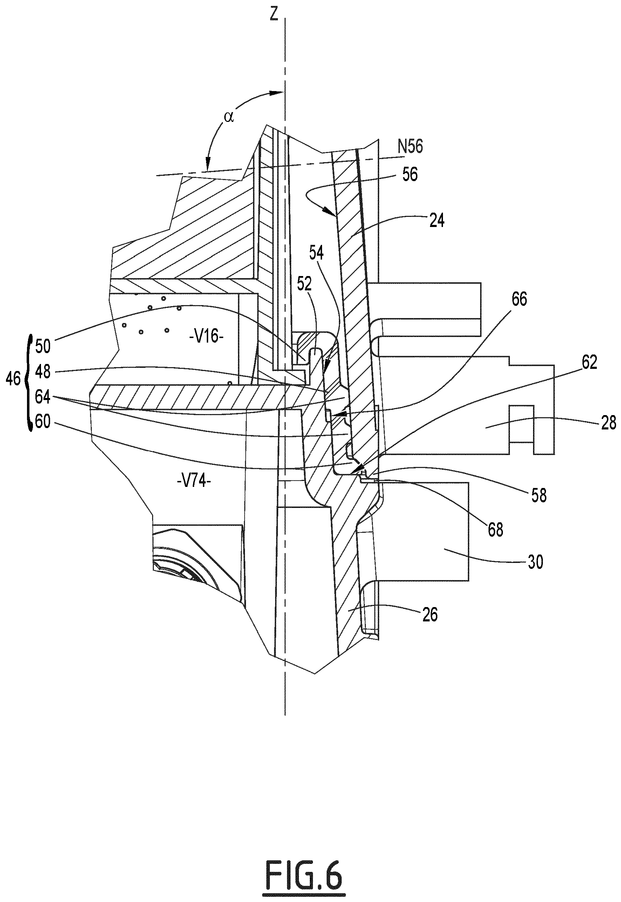

En outre, comme mieux visible à la figure 6, la coque supérieure 24 s'étend selon l'axe principal Z au-delà du joint d'étanchéité 46, de sorte à recouvrir le joint d'étanchéité. En d'autres termes, une extrémité 58 de la coque supérieure est située plus basse que le joint d'étanchéité 46, selon l'axe principal Z. Ainsi, depuis l'extérieur du boîtier 16, le joint d'étanchéité 46 n'est pas directement visible. Il est ainsi protégé des projections directes susceptibles de le détériorer, améliorant ainsi son vieillissement.

Le joint d'étanchéité 46 présente plusieurs barrières successives empêchant aux poussières et à l'eau de pénétrer à l'intérieur du boîtier 16.

La première de ces barrières est formée par une barrière anti-poussières 60, qui forme une extrémité basse de la paroi principale 58 du joint d'étanchéité 46, selon l'axe principal Z, et qui est comprimée entre l'extrémité 58 de la coque supérieure 24 et une face d'appui 62 de la coque inférieure 26, la face d'appui 62 étant de préférence perpendiculaire à l'axe principal Z.

La deuxième de ces barrières est formée par au moins une barrière anti-infiltrations 64, dans l'exemple par deux barrières anti-infiltrations 64. Les barrières anti-infiltrations sont formées sur la paroi principale 48 du joint d'étanchéité 46 et s'étendent depuis la paroi principale 48 vers la face intérieure 56 de la coque supérieure 24. Chaque barrière anti-infiltrations est comprimée entre la face extérieure 54 de la coque inférieure 26 et la face intérieure 56 de la coque supérieure 24. Sur la figure 6, les barrières anti-infiltrations 64 sont représentées dans leur état non-déformé.

Avantageusement, la troisième de ces barrières est formée par une cavité anti-capillarité 66, qui est aménagée entre la paroi principale 48 du joint d'étanchéité 46 et la bordure supérieure 52 de la coque inférieure 26.

La barrière anti-poussières 60, les barrières anti-infiltrations 64 et la cavité anti-capillarité 66 coopèrent ensemble pour éviter les infiltrations d'eau et de poussière entre la coque inférieure 26 et la coque supérieure 28 vers l'intérieur du boîtier 16.

Chaque barrière anti-infiltrations 64 se comporte comme un joint torique et est particulièrement efficace pour assurer l'étanchéité à l'eau de l'ouverture du boîtier 16 formée entre la coque supérieure 24 et la coque inférieure 26. Ainsi, la surface de contact entre chaque barrière anti-infiltrations 64 et la face intérieure 56 de la coque supérieure 24 est faible, et s'apparente à une surface de contact quasi-linéaire. Cette surface de contact faible induit une pression de contact élevée, ce qui est particulièrement efficace pour assurer l'étanchéité à l'eau. La compression de chaque barrière anti-infiltrations 64, lorsque le dispositif de surveillance 12 est assemblé, est comprise entre 15% et 35%, de préférence égale à 25%.

La barrière anti-poussières 60, quant à elle, présente une surface de contact importante avec l'extrémité 58 de la coque supérieure 24, d'une part, et avec la face d'appui 62 de la coque inférieure 26, d'autre part. Cette surface de contact importante rend la barrière anti-poussières 60 particulièrement adaptée pour empêcher le passage de poussières entre la coque supérieure 24 et la coque inférieure 26. En effet, la surface de contact importante fait que la distance à parcourir entre le joint et le boîtier par une particule de poussière est élevée, ce qui maximise les chances de retenir la particule de poussière au niveau de la barrière anti-poussières. En outre, les particules de poussières étant généralement de dimensions supérieures aux molécules ou gouttelettes d'eau, une pression de contact importante n'est pas requise pour les arrêter. La compression de la barrière anti-poussières 60, lorsque le dispositif de surveillance 12 est assemblé, est comprise entre 5% et 25%, de préférence égale à 15%.

En pratique, la barrière anti-poussières 60 et les barrières anti-infiltrations 64 participent toutes à la fois à l'étanchéité aux poussières et à l'étanchéité à l'eau, mais la barrière anti-poussières est particulièrement efficace pour empêcher les infiltrations de poussière, grâce à une surface de contact importante avec les coques supérieure 24 et inférieure 26, et les barrières anti-infiltrations sont particulièrement efficaces pour empêcher les infiltrations d'eau, grâce à une pression de contact importante avec la coque supérieure.

En outre, le fait que la barrière anti-poussières 60 est comprimée entre l'extrémité 58 de la coque supérieure 24 et la face d'appui 62 de la coque inférieure 26 est particulièrement avantageux pour garantir une bonne compression de la barrière anti-poussières grâce au système de fermeture. En effet, les organes de manoeuvre 32 permettent d'exercer une force importante sur les coques supérieure 24 et inférieure 26, selon l'axe principal Z, ce qui permet d'obtenir une compression de la barrière anti-poussières qu'il serait impossible à obtenir par un serrage manuel direct, sans le système de fermeture décrit ci-dessus. Le système de fermeture est donc particulièrement avantageux pour améliorer les performances du joint d'étanchéité 46.

De manière similaire, lorsqu'il est nécessaire d'ouvrir le boîtier 16, les organes de manoeuvre 32 permettent d'exercer une force importante de séparation des coques supérieure 24 et inférieur 26. Cette force importante est particulièrement avantageuse lorsque le dispositif de surveillance 12 est resté en place au sein de l'installation I pendant une durée importante, car après une durée importante, le joint d'étanchéité 46 tend à coller à la coque supérieure, et cette force permet alors de décoller la coque supérieure du joint d'étanchéité.

Avantageusement, la hauteur H46 du joint d'étanchéité 46 est comprise entre 10 mm et 40 mm. Dans l'exemple, la hauteur H46 est égale à 17 mm. En pratique, la hauteur H46 du joint d'étanchéité 46 est supérieure à la hauteur H1, de sorte que lorsque la coque supérieure 24 est positionnée sur la coque inférieure 26 avant le serrage, la coque supérieure entoure une partie du joint d'étanchéité, ce qui permet d'assurer un bon positionnement de la coque supérieure par rapport au joint d'étanchéité. Ainsi, le serrage des coques s'effectue ensuite sans risque de dégradation du joint d'étanchéité, grâce au bon positionnement de la coque supérieure, ce qui permet en outre d'assurer la bonne compression du joint d'étanchéité après fermeture du boîtier 16.

Les barrières anti-infiltrations 64 étant disposées sur la paroi principale 48 du joint d'étanchéité 46 du côté de la coque supérieure 24, des infiltrations d'eau peuvent se produire par capillarité le long de la paroi principale, du côté de la coque inférieure 26. La cavité anti-capillarité 66 est particulièrement avantageuse pour ralentir ces infiltrations par capillarité, en formant une réserve d'eau bloquant ce phénomène de capillarité. En effet, le volume d'eau susceptible de remonter le long du joint d'étanchéité 46 par capillarité est très faible, et la cavité anti-capillarité 66 présente un volume suffisant pour accueillir ce volume d'eau et empêcher la remontée d'eau jusqu'au volume intérieur V16 du boîtier, même lorsque le dispositif de surveillance 12 est immergé pendant une longue durée.

Avantageusement, la coque inférieure 26 du boîtier 16 présente des butées 68, permettant d'éviter une compression trop importante du joint d'étanchéité 46 et permettant de contrôler la position relative des coques supérieure 24 et inférieure 26, perpendiculairement à l'axe principal Z. En effet, sans ces butées, la coque supérieure 24 serait en mesure de présenter une légère rotation autour de l'axe X28, puisque les organes de manoeuvre 32 exercent un effort sur les coques supérieure 24 et inférieure 26 dans un plan formé par les axes X28 et Z, ce qui pourrait conduire à une compression non-uniforme du joint d'étanchéité 46, selon un axe perpendiculaire à l'axe X28 et à l'axe principal Z. Grâce aux butées 68, la coplanarité des coques supérieure 24 et inférieure 26 est assurée. En pratique, la coque inférieure 26 présente quatre butées 68, deux butées étant situées de part et d'autre de chacun des deux ergots inférieurs 30.

Avantageusement, le dispositif de surveillance 12 comprend un carter 69, qui entoure la carte électronique 18 de sorte à protéger la carte électronique des chocs lorsque le dispositif de surveillance est démonté. Le carter 69 n'est représenté que sur la figure 5. Le carter 69 est fixé à la bordure supérieure 52 de la coque inférieure 26 et s'étend depuis la coque inférieure selon l'axe principal Z. Lorsque le boîtier 16 est assemblé grâce au système de fermeture, le carter 69 guide le mouvement de la coque supérieure 24 par rapport à la coque inférieure 26, car la partie haute du carter est en contact avec la coque supérieure 24, qui glisse sur le carter lors de la fermeture du boîtier. Le carter 69 permet donc à la fois de protéger la carte électronique 18 et d'assurer un bon positionnement relatif des coques supérieure 24 et inférieure 26.

Comme mieux visible sur la figure 3, les éléments de connexion 22 sont fixés sur une extension 70 de la coque inférieure 26, qui s'étend selon l'axe principal Z en s'éloignant de la coque supérieure 24. L'extension présente une paroi d'extrémité 72, qui correspond à extrémité basse, c'est-à-dire son extrémité la plus éloignée de la coque supérieure 24, selon l'axe principal Z. Dans l'exemple, l'extension 70 présente une forme essentiellement parallélépipédique, et la paroi d'extrémité 72 est plane et perpendiculaire à l'axe principal Z.

L'intérieur de l'extension 70 fait partie du volume intérieur V16 du boîtier 16. En d'autres termes, l'extension 70 délimite la partie basse du volume intérieur V16. Les éléments de connexion 22 traversent l'extension 70 de sorte à permettre le raccordement des capteurs ou actionneurs 14 à la carte électronique 18.

Avantageusement, pour éviter les infiltrations d'eau de l'extérieur du boîtier 16 vers le volume intérieur V16 au niveau des éléments de connexion 22, la coque inférieure 26 présente une jupe 74, qui s'étend selon l'axe principal Z en s'éloignant de la coque supérieure 24 en entourant l'extension 70. La jupe 74 s'étend, selon l'axe principal Z, au moins jusqu'à la paroi d'extrémité 72 de l'extension 70. Autrement dit, la jupe 74 descend au moins jusqu'au niveau de la paroi d'extrémité 72. Dans l'exemple, la jupe 74 descend jusqu'au niveau de la paroi d'extrémité 72. En variante non-représentée de l'invention, la jupe 74 descend plus bas que la paroi d'extrémité 72.

Comme visible sur les figures 3 et 4, la jupe 74 et l'extension 70 forment entre elles un volume complémentaire d'étanchéité V74, désigné par la suite en tant que volume d'étanchéité V74 dans lequel les éléments de connexion 22 sont disposés. Plus précisément, les éléments de connexion 22 traversent l'extension 70, une partie des éléments de connexion est située dans le volume intérieur V16 du boîtier 16 et une partie des éléments de connexion est située dans le volume d'étanchéité V74.

En pratique, le volume d'étanchéité V74 est délimité, dans un plan perpendiculaire à l'axe principal Z, par la jupe 74, l'extension 70 étant située dans le volume d'étanchéité, et le volume d'étanchéité est délimité, selon l'axe principal Z vers le haut, par une paroi interne 76 de la coque inférieure 26. La paroi interne 76 délimite donc le volume d'étanchéité V74 du volume intérieur V16, selon l'axe principal Z. Dans un plan perpendiculaire à l'axe principal Z, au niveau de l'extension 70, l'extension délimite le volume d'étanchéité V74 du volume intérieur V16. En outre, le volume d'étanchéité V74 est ouvert, selon l'axe principal Z et vers le bas. Ainsi, les capteurs ou actionneurs 14 peuvent pénétrer librement le volume d'étanchéité V74 pour être connectés aux éléments de connexion 22.

Puisque l'axe principal Z est vertical et la coque supérieure 24 est située au-dessus de la coque inférieure 26 lorsque le dispositif de surveillance 12 est fixé au support 10, le volume d'étanchéité V74 forme une bulle d'air entourant les éléments de connexion 22. Ainsi, en cas de submersion du dispositif de surveillance 12, par exemple en cas de fortes pluies, la bulle d'air formée dans le volume V74 reste captive, car bloquée par la jupe 74 et la paroi interne 76 de la coque inférieure 26. Dès lors, l'immersion du dispositif de surveillance 12 n'entraîne pas le remplissage du volume d'étanchéité V74 avec de l'eau. Autrement dit, la bulle d'air comprise dans le volume V74 empêche la montée de l'eau dans le volume V74.

La jupe 74 est ainsi particulièrement avantageuse, car elle protège les éléments de connexion 22 en empêchant leur immersion, même lorsque le dispositif de surveillance 12 est immergé. Ainsi, l'étanchéité du dispositif de surveillance 12 est améliorée, car l'eau est empêchée de pénétrer dans le boîtier 16 par les éléments de connexion 22.

Dans l'exemple, le dimensionnement de la jupe 74 permet d'obtenir une bulle d'air dans le volume d'étanchéité V74 suffisamment importante pour éviter la montée de l'eau jusqu'aux éléments de connexion 22 lorsque le dispositif de surveillance 12 est immergé dans une colonne d'eau allant jusqu'à 4 mètres de hauteur. Ici, pour atteindre ces performances, une hauteur H74, qui correspond à la hauteur entre le bas de la jupe 74 et le bas des éléments de connexion 22, est au moins égale à 25 mm. En variante non-représentée de l'invention, d'autres dimensionnements de la jupe 74 sont possibles, calculés en fonction de la pression exercée par l'eau sur l'air du volume d'étanchéité V74.

Le joint d'étanchéité 46 et la jupe 74 coopèrent ensemble pour améliorer l'étanchéité du dispositif de surveillance 12, ce qui permet d'atteindre un indice de protection au moins égal à IP65, tel que défini par la norme CEI 60529 édition 2.1 de 2001 de la Commission électrotechnique internationale. Dans l'exemple, le dispositif de surveillance 12 présente un indice de protection IP68, c'est-à-dire qu'il est totalement protégé contre les poussières et qu'il est entièrement submersible au-delà de 1 mètre. Des essais en laboratoire ont permis de montrer que le dispositif de surveillance 12 tel que décrit dans l'exemple est étanche lorsqu'il est submergé à 4 mètres de profondeur pendant 30 jours. Ainsi, les éléments disposés à l'intérieur du boîtier 16, et notamment la carte électronique 18, sont efficacement protégés des infiltrations d'eau.

Pour permettre la fixation du dispositif de surveillance 12 au support 10, la jupe 74 présente des moyens d'accrochage 78, qui sont prévus pour coopérer avec des crochets 80 du support 10. En pratique, les moyens d'accrochage 78 viennent s'accrocher sur les crochets 80 et empêchent la translation du dispositif de surveillance 12 par rapport au support 10 perpendiculairement à l'axe principal Z, et empêchent la translation du dispositif de surveillance par rapport au support selon l'axe principal Z vers le bas, c'est-à-dire dans une direction Z1 parallèle à l'axe principal Z est orientée de la coque supérieure 24 vers la coque inférieure 26.

De préférence, pour accrocher le dispositif de surveillance 12 sur le support 10, le dispositif de surveillance est approché du support de sorte à positionner les moyens d'accrochage 78 au-dessus des crochets 80, puis le dispositif de surveillance est déplacé dans la direction Z1 de sorte à accrocher les moyens d'accrochage sur les crochets.

Il est particulièrement avantageux que le dispositif de surveillance 12 soit fixé au support 10 par les moyens d'accrochage 78 de la jupe 74. En effet, puisque le dispositif de surveillance 12 est fixé au support 10 grâce à la jupe 74, le positionnement de la jupe par rapport au support est précisément maîtrisé. Ainsi, il est simple de contrôler que l'extrémité basse de la jupe 74 est horizontale, ce qui assure que la bulle d'air contenue dans le volume d'étanchéité V74 occupe le plus grand volume possible lorsque le dispositif de surveillance 12 est immergé, permettant ainsi d'assurer une étanchéité optimale au niveau des éléments de connexion 22.

Avantageusement, la coque supérieure 24 présente des moyens de blocage 82 qui coopèrent avec une languette 84 du support 10 de sorte à empêcher une translation du dispositif de surveillance 12, par rapport au support, verticalement vers le haut, c'est-à-dire dans une direction Z2 parallèle à l'axe principal Z est orientée de la coque inférieure 26 vers la coque supérieure 24.

Ici, les moyens de blocage 82 sont une encoche formée sur la coque supérieure 24 contre laquelle vient prendre appui la languette 84. En pratique, pendant la fixation du dispositif de surveillance 12 sur le support 10, la languette vient se déformer contre la coque supérieure 24 lorsque le dispositif de surveillance est déplacé dans la direction Z1 jusqu'à la position d'accrochage des moyens d'accrochage 78 sur les crochets 80, et la languette 84 vient s'engager dans l'encoche 82 lorsque les moyens d'accrochage sont accrochés aux crochets. Dans l'exemple, pour démonter le dispositif de surveillance 12 du support 10, il suffit d'appliquer un effort sur la languette 84 de sorte à la désengager de l'encoche 82, puis de translater le dispositif de surveillance selon la direction Z2.

Avantageusement, le support 10 présente deux éperons 86. Lorsque le dispositif de surveillance 12 est fixé au support 10, chaque éperon 86 s'étend dans la cavité de réception 40 de l'un des deux organes de manoeuvre 32. Cette fixation des organes de manoeuvre sur le support 10 est particulièrement avantageuse, pour deux raisons.

D'une part, cette fixation des organes de manœuvre 32 sur les éperons 86 empêche d'accrocher le dispositif de surveillance 12 au support 10 lorsque les organes de manœuvre 32 ne sont pas en position de verrouillage, car dans ce cas, les éperons 86 viennent buter contre les organes de manœuvre. Ainsi, il est certain que le dispositif de surveillance est correctement fermé lorsqu'il est accroché au support.

D'autre part, cette fixation des organes de manœuvre 32 sur les éperons 86 empêche de mettre en place la poignée 38 lorsque le dispositif de surveillance 12 est accroché au support 10, et, par conséquent, empêche l'ouverture du dispositif de surveillance tant que ce dernier est accroché au support, ce qui permet d'éviter toute ouverture accidentelle ou malveillante du dispositif de surveillance en dehors des ouvertures souhaitées lors d'opérations de maintenance.

On comprend que la fixation du dispositif de surveillance 12 sur le support 10 s'effectue sans outils, ce qui est particulièrement avantageux pour faciliter cette fixation. La durée nécessaire pour fixer et démonter le dispositif de surveillance du support est ainsi réduite.

Avantageusement, les organes de manœuvre 32 et la coque supérieure 24 présentent des œillets 88, qui permettent, lorsque les organes de manœuvre sont en position de verrouillage, le passage de scellés empêchant la manœuvre des organes de manœuvre vers leur position de déverrouillage. Ainsi, une ouverture malveillante du dispositif de détection 12 nécessite le retrait des scellés, ce qui permet de détecter une telle ouverture.

En variante non-représentée de l'invention, la jupe 74 n'entoure pas entièrement l'extension 70, mais s'étend depuis l'extension 70 autour des éléments de connexion 22. En d'autres termes, dans cette variante, le volume d'étanchéité V74 est délimité, perpendiculairement à l'axe principal Z, par la jupe 74 et par l'extension 70, plus précisément par la face de l'extension 70 portant les éléments de connexion 22.

En variante non-représentée de l'invention, les organes de manœuvre 32 sont fixés sur les ergots inférieurs 30, au lieu d'être fixés sur les ergots supérieurs 28, c'est-à-dire que le logement 34 de chaque organe de manœuvre reçoit un ergot inférieur, et la gorge 36 de chaque organe de manœuvre reçoit un ergot supérieur.

En variante non-représentée de l'invention, les coques supérieure 24 et inférieure 26 présentent un nombre différent d'ergots supérieurs 28 et 30, par exemple quatre ergots supérieurs et quatre ergots inférieurs. De préférence, parmi les quatre ergots supérieurs, ainsi que parmi les quatre ergots inférieurs, deux sont alignés selon un premier axe perpendiculaire à l'axe principal Z, et deux autre sont alignés selon un deuxième axe perpendiculaire à l'axe principal Z et parallèle au premier axe. Dans une telle variante, le dispositif de surveillance 12 comprend quatre organes de manœuvre 32, et, de préférence, la fermeture du boîtier 16 s'effectue à l'aide de deux poignées 38, chaque poignée manœuvrant deux organes de manœuvre alignés selon l'un des deux axes parmi le premier et le deuxième axe. Dans une telle variante, la présence de butée 68 n'est pas nécessaire pour assurer la coplanarité des coques supérieure 24 et inférieure 26.

En variante non-représentée de l'invention, le joint d'étanchéité 46 est accroché à la coque supérieure 24, au lieu d'être accroché à la coque inférieure 26. Dans une telle variante, de préférence, les coques supérieure et inférieure sont prévues de sorte à ce que les barrières anti-infiltrations 64 soient compressées entre une face intérieure de la coque inférieure et une face extérieure de la coque supérieure, de sorte que la barrière anti-poussières 60 soit compressée entre une face d'appui de la coque supérieure et une extrémité de la coque inférieure, et de sorte que le joint d'étanchéité soit recouvert par la coque inférieure lorsque le boîtier 16 est fermé.

En variante non-représentée de l'invention, le dispositif de surveillance 12 présente, en plus de la jupe 74, un élément d'étanchéité, tel qu'un joint, disposé autour des éléments de connexion 22 et permettant d'éviter l'entrée d'eau dans le boîtier 16 au niveau des éléments de connexion en cas d'immersion du dispositif d'étanchéité dans une colonne d'eau dont la hauteur serait supérieure à la hauteur considérée pour dimensionner la jupe 74.

En variante non-représentée de l'invention, la coque inférieure 26 ne présente pas de jupe 74. Dans une telle variante, l'étanchéité au niveau des éléments de connexion 22 est assurée par d'autres moyens, par exemple à l'aide de résine disposée dans le fond de la coque inférieure 26 de sorte à recouvrir les éléments de connexion 22, du côté du volume intérieur V16. Une telle variante est également utile dans le cas d'un dispositif de surveillance 12 ne comprenant pas de capteurs ou actionneurs 14 externes au boîtier 16, ou bien des capteurs reliés à la carte électronique 18 à l'aide d'une liaison sans fil. Dans un tel cas, les éléments de connexion 22 ne sont pas présents, et la jupe 74 n'est alors pas nécessaire.

En variante non-représentée de l'invention, le dispositif de surveillance 12 comprend un seul organe de manœuvre 32, un seul ergot supérieur 28 et un seul ergot inférieur 30. Dans une telle variante, le dispositif de surveillance 12 présente de préférence des moyens de guidage de la coque supérieure 24 par rapport à la coque inférieure 26 permettant une compression uniforme du joint d'étanchéité 46 lorsque le boîtier 16 est fermé à l'aide de l'organe de manœuvre.

## Revendications

1. Dispositif de surveillance (12), comprenant un boîtier (16) formant un volume intérieur (V16) et une carte électronique (18) disposée dans le volume intérieur du boîtier, le dispositif de surveillance (12) étant prévu pour être accroché sur un support (10), dans lequel le boîtier comporte une coque inférieure (26) et une coque supérieure (24), juxtaposées selon un axe principal (Z) du dispositif de surveillance, la coque supérieure étant assemblée sur la coque inférieure, le dispositif de surveillance (12) étant **caractérisé en ce qu'**il comprend :
- un joint d'étanchéité (46), s'étendant dans un plan perpendiculaire à l'axe principal (Z) entre la coque inférieure (26) et la coque supérieure (24),
- un système de fermeture, comportant :
∘ au moins un ergot inférieur (30) appartenant à la coque inférieure (26),
∘ au moins un ergot supérieur (28) appartenant à la coque supérieure (24),
∘ au moins un organe de manœuvre (32), le ou chaque organe de manœuvre coopérant avec un ergot inférieur (30) et avec un ergot supérieur (28),
et **en ce que** le ou chaque organe (32) de manœuvre est actionnables entre :
- une position de verrouillage, dans laquelle le dispositif de surveillance (12) est dans une configuration assemblée, la carte électronique (18) étant alors protégée par le boîtier (16), et
- une position de déverrouillage, dans laquelle le dispositif de surveillance (12) est dans une configuration non-assemblée, la carte électronique (18) étant alors accessible,
dans lequel l'actionnement de l'au moins un organe de manœuvre (32) de la position de déverrouillage vers la position de verrouillage engendre un rapprochement de l'au moins un ergot inférieur (30) et de l'au moins un ergot supérieur (28), entraînant une translation de la coque inférieure et de la coque supérieure l'une vers l'autre, selon l'axe principal (Z), et entraînant la compression du joint d'étanchéité (46) entre la coque supérieure et la coque inférieure.

2. Dispositif de surveillance (12) selon la revendication 1, dans lequel le ou chaque organe de manœuvre (32) comprend :
- un logement (34) de réception d'un premier ergot (28, 30) parmi l'ergot supérieur (28) et l'ergot inférieur (30) associés à cet organe de manœuvre, et
- une gorge (36) de guidage du second ergot (28, 30) parmi l'ergot supérieur (28) et l'ergot inférieur (30) associés à cet organe de manœuvre,
dans lequel le ou chaque organe de manœuvre (32) est actionnable en rotation autour du premier ergot (28) associé à cet organe de manœuvre,
et dans lequel le ou chaque organe de manœuvre (32) est configuré pour transformer un mouvement de rotation de l'organe de manœuvre en un mouvement de translation du second ergot (28, 30) associé à cet organe de manœuvre selon l'axe principal (Z).

3. Dispositif de surveillance (12) selon l'une des revendications 1 et 2, dans lequel le système de fermeture du dispositif de surveillance (12) comprend au moins deux ergots inférieurs (30), au moins deux ergots supérieurs (28) et au moins deux organes de manœuvre (32).

4. Dispositif de surveillance (12) selon la revendication 3, dans lequel le dispositif de surveillance (12) comprend deux organes de manœuvre (32) et une poignée (38) reliant les deux organes de manœuvre (32), et dans lequel l'actionnement de la poignée entraîne l'actionnement des deux organes de manœuvre.

5. Dispositif de surveillance (12) selon la revendication 4, dans lequel la poignée (38) est amovible, dans lequel chaque organe de manœuvre (32) présente une cavité de réception (40) de la poignée, et dans lequel la cavité de réception de chaque organe de manœuvre est configurée pour recevoir un éperon (86) appartenant au support (10), permettant l'accrochage du dispositif de surveillance (12) au support (10).

6. Dispositif de surveillance (12) selon l'une quelconque des revendications 1 à 5, dans lequel le joint d'étanchéité (46) comprend au moins une barrière anti-infiltrations (64) configurée pour empêcher l'infiltration d'eau entre la coque supérieure (24) et la coque inférieure (26), et comprend une barrière anti-poussières (60), configurée pour empêcher le passage de poussières entre la coque supérieure (24) et la coque inférieure (26).

7. Dispositif de surveillance (12) selon la revendication 6, dans lequel l'au moins une barrière anti-infiltrations (64) est comprimée entre une face extérieure (54) d'une première coque (24, 26) parmi la coque supérieure (24) et la coque inférieure (26), la face extérieure (54) étant dirigée à l'opposé du volume intérieur (V16) du boîtier (16), et une face intérieure (56) de la deuxième coque (24, 26) parmi la coque supérieure (24) et la coque inférieure (26), la face intérieure étant dirigée vers le volume intérieur du boîtier, et dans lequel la barrière anti-poussières (60) est comprimée entre une face d'appui (62) de la première coque et une extrémité (58) de la deuxième coque, la face d'appui étant perpendiculaire à l'axe principal (Z).

8. Dispositif de surveillance (12) selon la revendication 7, dans lequel la deuxième coque (24) recouvre le joint d'étanchéité (46).

9. Dispositif de surveillance (12) selon l'une quelconque des revendications 1 à 8, dans lequel le dispositif de surveillance comprend un ou plusieurs éléments de connexion (22), chaque élément de connexion étant fixé au boîtier et connecté à la carte électronique (18), et dans lequel la coque inférieure (26) comprend :
- une extension (70), s'étendant selon l'axe principal (Z) à l'opposé de la coque supérieure (24), l'extension délimitant le volume intérieur (V16) du boîtier (16), les éléments de connexion (22) étant fixés à l'extension (70), et
- une jupe (74), s'étendant selon l'axe principal (Z) à l'opposé de la coque supérieure, la jupe s'étendant au moins jusqu'à une paroi d'extrémité (72) de l'extension (70), selon l'axe principal (Z), la jupe entourant les éléments de connexion (22) fixés à l'extension (70),
et dans lequel l'extension (70) et la jupe (74) forment entre elles un volume complémentaire d'étanchéité (V74), les éléments de connexion (22) étant disposés dans le volume complémentaire d'étanchéité.

10. Dispositif de surveillance (12) selon la revendication 9, dans lequel la jupe (74) présente des moyens d'accrochage (78), les moyens d'accrochage étant configurés pour permettre l'accrochage du dispositif de surveillance (12) au support (10) et pour :
- empêcher une translation du dispositif de surveillance (12), par rapport au support (10), perpendiculairement à l'axe principal (Z), et
- empêcher une translation du dispositif de surveillance (12), par rapport au support, dans une direction (Z1) parallèle à l'axe principal et orientée de la coque supérieure (24) vers la coque inférieure (26),
et dans lequel, de préférence, le dispositif de surveillance (12) est configuré pour que l'accrochage du dispositif de surveillance sur le support (10) ne soit possible que lorsque les organes de manoeuvre (32) sont en position de verrouillage.

11. Dispositif de surveillance (12) selon la revendication 10, dans lequel la coque supérieure (24) présente des moyens de blocage (82), configurés pour coopérer avec le support (10) de sorte à empêcher une translation du dispositif de surveillance (12), par rapport au support, dans une direction (Z2) parallèle à l'axe principal (Z) et orientée de la coque inférieure (26) vers la coque supérieure (24).

## Patentansprüche

1. Überwachungsvorrichtung (12), umfassend ein Gehäuse (16), das ein Innenvolumen (V16) bildet, und eine elektronische Leiterplatte (18), die im Innenvolumen des Gehäuses angeordnet ist, wobei die Überwachungsvorrichtung (12) dazu vorgesehen ist, an einem Träger (10) aufgehängt zu werden, wobei das Gehäuse eine untere Gehäuseschale (26) und eine obere Gehäuseschale (24) aufweist, die entlang einer Hauptachse (Z) der Überwachungsvorrichtung aneinandergefügt sind, wobei die obere Gehäuseschale auf der unteren Gehäuseschale montiert ist, wobei die Überwachungsvorrichtung (12) **dadurch gekennzeichnet ist, dass** sie umfasst:
- eine Dichtung (46), die sich in einer zur Hauptachse (Z) senkrechten Ebene erstreckt zwischen der unteren Gehäuseschale (26) und der oberen Gehäuseschale (24),
- ein Verschlusssystem, aufweisend:
∘ mindestens einen unteren Zapfen (30), der zur unteren Gehäuseschale (26) gehört,
∘ mindestens einen oberen Zapfen (28), der zur oberen Gehäuseschale (24) gehört,
∘ mindestens ein Betätigungselement (32), wobei das oder jedes Betätigungselement mit einem unteren Zapfen (30) und mit einem oberen Zapfen (28) zusammenwirkt,
und dass das oder jedes Betätigungselement (32) betätigbar ist zwischen:
- einer Verriegelungsstellung, in der sich die Überwachungsvorrichtung (12) in einer zusammengefügten Konfiguration befindet, wobei die elektronische Leiterplatte (18) dann durch das Gehäuse (16) geschützt ist, und
- einer Entriegelungsstellung, in der sich die Überwachungsvorrichtung (12) in einer nicht zusammengefügten Konfiguration befindet, wobei die elektronische Leiterplatte (18) dann zugänglich ist,
wobei die Betätigung des mindestens einen Betätigungselements (32) von der Entriegelungsstellung in die Verriegelungsstellung eine Annäherung des mindestens einen unteren Zapfens (30) und des mindestens einen oberen Zapfens (28) bewirkt, was eine Verschiebung der unteren Gehäuseschale und der oberen Gehäuseschale aufeinander zu entlang der Hauptachse (Z) und eine Kompression der Dichtung (46) zwischen der oberen Gehäuseschale und der unteren Gehäuseschale bewirkt.

2. Überwachungsvorrichtung (12) nach Anspruch 1, wobei das oder jedes Betätigungselement (32) umfasst: :
- eine Aufnahme (34) zur Aufnahme eines ersten Zapfens (28, 30) aus dem oberen Zapfen
(28) und dem unteren Zapfen (30), die diesem Betätigungselement zugeordnet sind, und
- eine Nut (36) zur Führung des zweiten Zapfens (28, 30) aus dem oberen Zapfen (28) und dem unteren Zapfen (30), die diesem Betätigungselement zugeordnet sind,
wobei das oder jedes Betätigungselement (32) um den diesem Betätigungselement zugeordneten ersten Zapfen (28) drehbar betätigbar ist,
und wobei das oder jedes Betätigungselement (32) dazu ausgebildet ist, eine Drehbewegung des Betätigungselements in eine Verschiebebewegung des diesem Betätigungselement zugeordneten zweiten Zapfens (28, 30) entlang der Hauptachse (Z) umzuwandeln.

3. Überwachungsvorrichtung (12) nach einem der Ansprüche 1 und 2, wobei das Verschlusssystem der Überwachungsvorrichtung (12) mindestens zwei untere Zapfen (30), mindestens zwei obere Zapfen (28) und mindestens zwei Betätigungselemente (32) umfasst.

4. Überwachungsvorrichtung (12) nach Anspruch 3, wobei die Überwachungsvorrichtung (12) zwei Betätigungselemente (32) und einen Griff umfasst
(38), der die beiden Betätigungselemente (32) verbindet, und wobei die Betätigung des Griffs die Betätigung der beiden Betätigungselemente bewirkt.

5. Überwachungsvorrichtung (12) nach Anspruch 4, wobei der Griff (38) abnehmbar ist, wobei jedes Betätigungselement (32) einen Aufnahmehohlraum (40) für den Griff aufweist, und wobei der Aufnahmehohlraum jedes Betätigungselements dazu ausgebildet ist, einen zum Träger (10) gehörenden Dorn (86) aufzunehmen, wodurch das Aufhängen der Überwachungsvorrichtung (12) am Träger (10) ermöglicht wird.

6. Überwachungsvorrichtung (12) nach einem der Ansprüche 1 bis 5, wobei die Dichtung (46) mindestens eine Infiltrationssperre (64) umfasst, die dazu ausgebildet ist, das Eindringen von Wasser zwischen der oberen Gehäuseschale (24) und der unteren Gehäuseschale (26) zu verhindern, und eine Staubsperre (60) umfasst, die dazu ausgebildet ist, den Durchtritt von Staub zwischen der oberen Gehäuseschale (24) und der unteren Gehäuseschale (26) zu verhindern.

7. Überwachungsvorrichtung (12) nach Anspruch 6, wobei die mindestens eine Infiltrationssperre (64) zwischen einer Außenfläche (54) einer ersten Gehäuseschale (24, 26) aus der oberen Gehäuseschale (24) und der unteren Gehäuseschale (26), wobei die Außenfläche (54) vom Innenvolumen (V16) des Gehäuses (16) weggerichtet ist, und einer Innenfläche (56) der zweiten Gehäuseschale (24, 26) aus der oberen Gehäuseschale (24) und der unteren Gehäuseschale (26), wobei die Innenfläche zum Innenvolumen des Gehäuses gerichtet ist, komprimiert ist, und wobei die Staubsperre (60) zwischen einer Anlagefläche (62) der ersten Gehäuseschale und einem Ende (58) der zweiten Gehäuseschale komprimiert ist, wobei die Anlagefläche senkrecht zur Hauptachse (Z) ist.

8. Überwachungsvorrichtung (12) nach Anspruch 7, wobei die zweite Gehäuseschale (24) die Dichtung (46) überdeckt.

9. Überwachungsvorrichtung (12) nach einem der Ansprüche 1 bis 8, wobei die Überwachungsvorrichtung ein oder mehrere Verbindungselemente (22) umfasst, wobei jedes Verbindungselement am Gehäuse befestigt und mit der
elektronischen Leiterplatte (18) verbunden ist, und wobei die untere Gehäuseschale (26) umfasst:
- eine Verlängerung (70), die sich entlang der Hauptachse (Z) von der oberen Gehäuseschale (24) weg erstreckt, wobei die Verlängerung das Innenvolumen (V16) des Gehäuses (16) begrenzt, wobei die Verbindungselemente (22) an der Verlängerung (70) befestigt sind, und
- eine Schürze (74), die sich entlang der Hauptachse (Z) von der oberen Gehäuseschale weg erstreckt, wobei sich die Schürze entlang der Hauptachse (Z) mindestens bis zu einer Endwand (72) der Verlängerung (70) erstreckt, wobei die Schürze die an der Verlängerung (70) befestigten Verbindungselemente (22) umgibt,
und wobei die Verlängerung (70) und die Schürze (74) zwischen sich ein ergänzendes Abdichtungsvolumen (V74) bilden, wobei die Verbindungselemente (22) im ergänzenden Abdichtungsvolumen angeordnet sind.

10. Überwachungsvorrichtung (12) nach Anspruch 9, wobei die Schürze (74) Aufhängemittel (78) aufweist, wobei die Aufhängemittel dazu ausgebildet sind, das Aufhängen der Überwachungsvorrichtung (12) am Träger (10) zu ermöglichen und :
- eine Verschiebung der Überwachungsvorrichtung (12) relativ zum Träger (10) senkrecht zur Hauptachse (Z) zu verhindern, und
- eine Translation der Überwachungsvorrichtung (12) relativ zum Träger in einer Richtung (Z1), die parallel zur Hauptachse verläuft und von der oberen Gehäuseschale (24) zur unteren Gehäuseschale (26) ausgerichtet ist, zu verhindern,
und wobei vorzugsweise die Überwachungsvorrichtung (12) so konfiguriert ist, dass das Einhängen der Überwachungsvorrichtung an dem Träger (10) nur möglich ist, wenn sich die Betätigungselemente (32) in der Verriegelungsposition befinden.

11. Überwachungsvorrichtung (12) nach Anspruch 10, bei der die obere Gehäuseschale (24) Blockiermittel (82) aufweist, die dazu konfiguriert sind, mit dem Träger (10) zusammenzuwirken, um eine Translation der Überwachungsvorrichtung (12) relativ zum Träger in einer Richtung (Z2), die parallel zur Hauptachse (Z) verläuft und von der unteren Gehäuseschale (26) zur oberen Gehäuseschale (24) ausgerichtet ist, zu verhindern.

## Claims

1. A monitoring device (12), comprising a housing (16) forming an interior volume (V16) and an electronic board (18) disposed within the interior volume of the housing, the monitoring device (12) being configured to be engaged on a support (10), wherein the housing includes a lower shell (26) and an upper shell (24), juxtaposed along a main axis (Z) of the monitoring device, the upper shell being assembled on the lower shell, the monitoring device (12) being **characterized in that** it comprises:
- a sealing gasket (46), extending in a plane perpendicular to the main axis (Z) between the lower shell (26) and the upper shell (24),
- a closure system, including:
∘ at least one lower lug (30) pertaining to the lower shell (26),
∘ at least one upper lug (28) pertaining to the upper shell (24),
∘ at least one maneuvering member (32), each maneuvering member cooperating with a lower lug (30) and with an upper lug (28),
wherein each maneuvering member (32) is operable between:
- a locking position, wherein the monitoring device (12) is in an assembled configuration, the electronic board (18) therefore being protected by the housing (16), and
- an unlocking position, wherein the monitoring device (12) is in a non-assembled configuration, the electronic board (18) therefore being accessible,
wherein the actuation of the at least one maneuvering member (32) from the unlocking position to the locking position causes a bringing together of the at least one lower lug (30) and the at least one upper lug (28), resulting in a translation of the lower shell and the upper shell towards each other, along the main axis (Z), and causing the compression of the sealing gasket (46) between the upper shell and the lower shell.

2. The monitoring device (12) according to claim 1, wherein each maneuvering member (32) comprises:
- a receiving housing (34) for a first lug (28, 30) among the upper lug (28) and the lower lug (30) associated with this maneuvering member, and
- a guide groove (36) for the second lug (28, 30) among the upper lug (28) and the lower lug (30) associated with this maneuvering member,
wherein each maneuvering member (32) is operable in rotation around the first lug (28) associated with this maneuvering member,
and wherein each maneuvering member (32) is configured to transform a rotational movement of the maneuvering member into a translational movement of the second lug (28, 30) associated with this maneuvering member along the main axis (Z).

3. The monitoring device (12) according to one of claims 1 and 2, wherein the closure system of the monitoring device (12) comprises at least two lower lugs (30), at least two upper lugs (28), and at least two maneuvering members (32).

4. The monitoring device (12) according to claim 3, wherein the monitoring device (12) comprises two maneuvering members (32) and a handle (38) connecting the two maneuvering members (32), and wherein the actuation of the handle causes the actuation of the two maneuvering members.

5. The monitoring device (12) according to claim 4, wherein the handle (38) is removable, wherein each maneuvering member (32) presents a receiving cavity (40) for the handle, and wherein the receiving cavity of each maneuvering member is configured to receive a spur (86) pertaining to the support (10), allowing the monitoring device (12) to be engaged on the support (10).

6. The monitoring device (12) according to any one of claims 1 to 5, wherein the sealing gasket (46) comprises at least one anti-infiltration barrier (64) configured to prevent water infiltration between the upper shell (24) and the lower shell (26), and comprises a dust barrier (60), configured to prevent the passage of dust between the upper shell (24) and the lower shell (26).

7. The monitoring device (12) according to claim 6, wherein the at least one anti-infiltration barrier (64) is compressed between an outer face (54) of a first shell (24, 26) among the upper shell (24) and the lower shell (26), the outer face (54) being directed opposite the interior volume (V16) of the housing (16), and an inner face (56) of the second shell (24, 26) among the upper shell (24) and the lower shell (26), the inner face being directed towards the interior volume of the housing, and wherein the dust barrier (60) is compressed between a bearing face (62) of the first shell and an end (58) of the second shell, the bearing face being perpendicular to the main axis (Z).

8. The monitoring device (12) according to claim 7, wherein the second shell (24) covers the sealing gasket (46).

9. The monitoring device (12) according to any one of claims 1 to 8, wherein the monitoring device comprises one or more connection elements (22), each connection element being fixed to the housing and connected to the electronic board (18), and wherein the lower shell (26) comprises:
- an extension (70), extending along the main axis (Z) opposite the upper shell (24), the extension delimiting the interior volume (V16) of the housing (16), the connection elements (22) being fixed to the extension (70), and
- a skirt (74), extending along the main axis (Z) opposite the upper shell, the skirt extending at least to an end wall (72) of the extension (70), along the main axis (Z), the skirt surrounding the connection elements (22) fixed to the extension (70),
and wherein the extension (70) and the skirt (74) therebetween form a complementary sealing volume (V74), the connection elements (22) being disposed within the complementary sealing volume.

10. The monitoring device (12) according to claim 9, wherein the skirt (74) presents attachment means (78), the attachment means being configured to allow the monitoring device (12) to be engaged on the support (10) and to:
- prevent a translation of the monitoring device (12), relative to the support (10), perpendicularly to the main axis (Z), and
- prevent a translation of the monitoring device (12), relative to the support, in a direction (Z1) parallel to the main axis and oriented from the upper shell (24) towards the lower shell (26),
and wherein, preferably, the monitoring device (12) is configured so that the monitoring device can only engage on the support (10) when the maneuvering members (32) are in the locking position.

11. The monitoring device (12) according to claim 10, wherein the upper shell (24) presents blocking means (82), configured to cooperate with the support (10) to prevent a translation of the monitoring device (12), relative to the support, in a direction (Z2) parallel to the main axis (Z) and oriented from the lower shell (26) towards the upper shell (24).
